(19) Europäisches Patentamt
European Patent Office
Office européen des brevets

(11) EP 1 635 791 B1

(12) **EUROPEAN PATENT SPECIFICATION**

(45) Date of publication and mention of the grant of the patent:
**02.07.2008 Bulletin 2008/27**

(21) Application number: **04735619.1**

(22) Date of filing: **01.06.2004**

(51) Int Cl.:
***A61K 9/20*** *(2006.01)* ***A61K 9/48*** *(2006.01)*
***A61K 31/40*** *(2006.01)* ***A61P 3/06*** *(2006.01)*
***A61P 19/10*** *(2006.01)* ***A61P 13/08*** *(2006.01)*

(86) International application number:
**PCT/IB2004/001845**

(87) International publication number:
**WO 2004/110409 (23.12.2004 Gazette 2004/52)**

(54) **PHARMACEUTICAL COMPOSITIONS COMPRISING ATORVASTATIN MANUFACTURED WITHOUT GRANULATION**

PHARMAZEUTISCHE ZUSAMMENSETZUNGEN MIT ATORVASTATIN, HERGESTELLT OHNE GRANULATION

COMPOSITIONS PHARMACEUTIQUES D'ATORVASTATINE, QUI SONT PRODUITES SANS PROCEDE DE GRANULATION

(84) Designated Contracting States:
**AT BE BG CH CY CZ DE DK EE ES FI FR GB GR HU IE IT LI LU MC NL PL PT RO SE SI SK TR**

(30) Priority: **12.06.2003 US 477918 P**

(43) Date of publication of application:
**22.03.2006 Bulletin 2006/12**

(60) Divisional application:
**08153178.2**

(73) Proprietor: **Warner-Lambert Company LLC**
**New York, NY 10017 (US)**

(72) Inventors:
- **BERCHIELLI, Alfred**
  **Groton, CT 06340 (US)**
- **WATERMAN, Kenneth Craig**
  **Groton, CT 06340 (US)**
- **DAUGHERITY, Patrick David**
  **Groton, CT 06340 (US)**

(74) Representative: **Hayles, James Richard**
**Pfizer Limited**
**European Patent Department**
**Ramsgate Road**
**Sandwich, Kent CT13 9NJ (GB)**

(56) References cited:
**EP-A- 1 336 405**
**WO-A-99/36060**
**WO-A-03/013608**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

CROSS-REFERENCE TO RELATED APPLICATIONS

This application claims priority from U.S. Provisional Patent Application No. 60/477,918 filed June 12,2003.

FIELD OF THE INVENTION

This invention relates to pharmaceutical compositions comprising atorvastatin and pharmaceutically acceptable salts thereof and a process for the preparation of the same, kits containing such compositions, as well as methods of using such compositions to treat subjects suffering from hypercholesterolemia and/or hyperlipidemia, as well as osteoporosis, benign prostatic hyperplasia (BPH), and Alzheimer's disease.

BACKGROUND OF THE INVENTION

The conversion of 3-hydroxy-3-methylglutaryl-coenzyme A (HMG-CoA) to mevalonate is an early and rate-limiting step in the cholesterol biosynthetic pathway. This step is catalyzed by the enzyme HMG-CoA reductase. Statins inhibit HMG-CoA reductase from catalyzing this conversion. As such, statins are collectively potent lipid lowering agents.

Atorvastatin calcium, disclosed in United States Patent No. 5,273,995 which is incorporated herein by reference, is currently sold as Lipitor® having the chemical name [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid calcium salt (2: 1) trihydrate and the formula

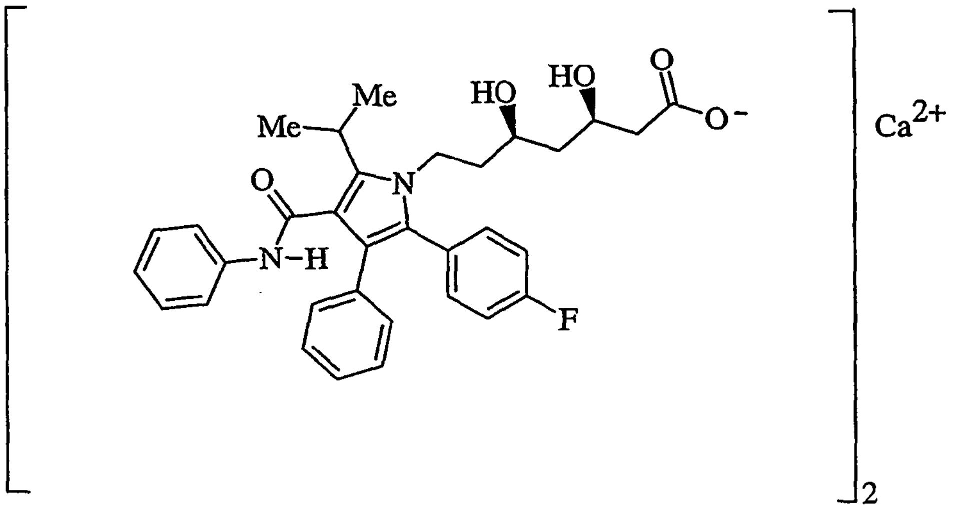

Atorvastatin and pharmaceutically acceptable salts thereof are selective, competitive inhibitors of HMG-CoA reductase. As such, atorvastatin calcium is a potent lipid-lowering compound and is thus useful as a hypolipidemic and/or hypocholesterolemic agent, as well as in the treatment of osteoporosis, benign prostatic hyperplasia (BPH), and Alzheimer's disease.

A number of patents have issued disclosing atorvastatin, formulations of atorvastatin, as well as processes and key intermediates for preparing atorvastatin. These include: United States Patent Numbers 4,681,893; 5,273,995; 5,003,080, 5,097,045; 5,103,024; 5,124,482; 5,149,837; 5,155,251; 5,216,174; 5,245,047; 5,248,793; 5,280,126; 5,397,792; 5,342,952; 5,298,627; 5,446,054; 5,470,981; 5,489,690; 5,489,691; 5,510,488; 5,686,104; 5,998,633; 6,087,511; 6,126,971; 6,433,213; and 6,476,235, which are herein incorporated by reference.

Atorvastatin can exist in crystalline, liquid crystalline and non-crystalline and amorphous forms.

Crystalline forms of atorvastatin calcium are disclosed in United States Patent Numbers 5,969,156 and 6,121,461, which are herein incorporated by reference. Further crystalline forms of atorvastatin are disclosed United States Patent 6,605,729 which is herein incorporated by reference.

Additionally, a number of published International Patent Applications have disclosed crystalline forms of atorvastatin, as well as processes for preparing amorphous atorvastatin. These include: WO 00/71116; WO 01/28999; WO 01/36384; WO 01/42209; WO 02/41834; WO 02/43667 WO 02/43732; WO 02/051804; WO 02/057228; WO 02/057229; WO 02/057274: WO 02/059087; WO 02/083637; WO 02/083638; WO 03/011826; WO 03/050085; WO 03/070702 and

WO 04/022053.

It has been disclosed that the amorphous forms in a number of drugs exhibit different dissolution characteristics and in some cases different bioavailability patterns compared to the crystalline form (Konno, T., Chem. Pharm. Bull., 1990;38:2003-2007). For some therapeutic indications one bioavailability pattern may be favored over another.

Variations in dissolution rates can make it advantageous to produce atorvastatin formulations in either crystalline or amorphous forms. For example, for some potential uses of atorvastatin (e.g., acute treatment of patients having strokes as described in Takemoto, M.; Node, K; Nakagami, H.; Liao, Y.; Grimm, M.; Takemoto, Y.; Kitakaze, M.; Liao, J.K., Journal of Clinical Investigation, 2001; 108(10): 1429-1437) a rapid onset of activity may be highly beneficial in improving the efficacy of the drug.

The preparation of solid formulations of atorvastatin is described in United States Patent Numbers 5,686,104 and 6,126,971. In the process described therein, atorvastatin is combined with a stabilizing additive, such as, an alkaline earth metal salt and excipients and subjected to wet granulation. US-A-2004/024767 discloses a wet granulated pharmaceutical composition of atorvastatin with less than about 5 weight% of an alkaline earth metal salt additive. Although wet granulation processes are widely used in the pharmaceutical industry, it is generally desirable to avoid wet granulations, if possible, since they add a process step, which decreases the overall manufacturing efficiency. Wet granulations, in general, are carried out to improve a number of properties of the drug plus excipients combination. Additionally, US-A-2004/0253305 discloses a dry-granulated pharmaceutical composition comprising atorvastatin.

Since atorvastatin is a highly potent drug, formulations of the drug are generally quite dilute in order to provide dosage forms of adequate size for manufacturing and patient handling. When a drug is used in a dilute form, the danger exists that segregation between the drug and excipients, during the processes before the drug is in its final dosage form, could lead to some of the unit dosage forms being hypo or hyperpotent. Potency control of the unit dosage forms is essential to prevent individual patients from receiving an incorrect, and sub-therapeutic or side-effect generating dose of the drug. Granulations are one method for preventing segregation. No method currently exists to predict, *a priori*, which, if any, excipients will provide sufficiently low segregation in combination with a given drug to enable that excipient to be used in a process for production of unit dosage forms without a granulation step. Therefore, there remains a need to identify atorvastatin plus excipient compositions suitable for preparation of unit dosage forms wherein the preparation therein does not involve a granulation step, and segregation is minimal. In the present invention, we have found that excipients appropriate for use in the preparation of unit dosage forms of atorvastatin without the need for a granulation step can unexpectedly be identified by a simple test procedure.

Within the group of excipients needed to provide an advantageous formulation of atorvastatin, the bulk excipient or excipient combination serves to dilute the drug and thereby provide a convenient amount of a unit dosage form for manufacturing and handling. Such materials are known in the pharmaceutical field as diluents. Diluents are described, for example, in "Handbook of Pharmaceutical Excipients, 3rd Edition" (A. H. Kibbe, Editor; Pharmaceutical Press, London; 2000). Since these materials comprise the bulk of formulations of atorvastatin, there remains a need to identify diluents that provide for good dose uniformity for production of unit dosage forms under commercial production conditions.

International Patent Applications WO 00/35425 and WO 01/76566A1 disclose formulations for formation of unit dosage forms of atorvastatin as part of a broader discussion of methods of stabilizing statin drugs. These disclosures do not, however, address uniformity of the drug doses delivered nor the suitability of such formulations for use with commercially viable processes. In fact, the formulations disclosed involve excipients that are available in a range of particle sizes, many of which do not allow for the tight weight and potency control needed for commercial production.

Additionally, in preparation and storage of unit dosage forms of atorvastatin, it is important to provide the active drug in a pure form. Moreover, it is desirable to achieve this high purity and stability with as simple a formulation as possible. United States Patent numbers 5,686,104 and 6,126,971 disclose a method for stabilization of atorvastatin formulations using alkaline earth metal salt additives in a wet granulation process. There remains a need to provide simple formulations and processes for preparation of unit dosage forms of atorvastatin which have low levels of impurities and provide adequate stability to allow dosage form expiration times that are commercially viable. In addition, alkaline earth metal salt additives and other alkalizing agent additives in compositions and dosage forms can affect drug dissolution potentially affecting the pharmacokinetics of the drug *in vivo*. It therefore remains desirable to provide atorvastatin formulations suitable for preparation of unit dosage forms whereby adequate drug purity, stability, and desired dissolution rate and bioavailability is provided with minimal levels of these materials.

WO9936060 discloses a pharmaceutical composition comprising excipients and atorvastatin as one of many possible active agents. This composition was manufactured by mixing, blending, milling and finally filled into a capsule shell. It did not contain a step of granulation. Sufficient base is added to establish the solution at a pH of at least 9.0, i.e. an alkalizing agent is added.

In WO03013608 a pharmaceutical composition in the form of a capsule is described which comprises an effective amount of at least one peroxisome proliferator activated agent (PPARalpha=peroxisome proliferator activated receptor alpha), an effective amount of one HMG-CoA reductase inhibitor derivative of the statin family (such as atorvastatin), at least one polyglycolized glyceride or another derivative of glycerides as well as other excipients. This composition is used for the manufacture of a medicament for treating hyperlipidaemia and hypercholesterolaemia. The process of manufacturing did not include a step of granulation.

EP1336405 published on 20.08.2003 (priority date of 14.02.2002) discloses pharmaceutical compositions (tablet, capsule) comprising amorphous atorvastatin, diluents and other excipients. 1.2-5 % alkali metal salt additives may be added to said mixture. The method of manufacturing this pharmaceutical composition does not contain a granulation step and includes the reduction of the particle size of amorphous atorvastatin by milling, the formation of mixture with the other excipients and the compression of this mixture into a pharmaceutical dosage form. This pharmaceutical composition is used for the manufacture of a medicament to treat dyslipoproteinaemia and hypercholesterolaemia.

Overall, it is an object of the present invention to provide acceptable potency variability between unit dosage forms of atorvastatin produced in high-speed production equipment using formulations and processes that provide stable dosage forms of atorvastatin. It is a further object of the present invention to provide consistency and stability with non-crystalline or amorphous forms of atorvastatin.

SUMMARY OF THE INVENTION

Accordingly, the first aspect of the present invention is a unit dosage form comprising atorvastatin or a pharmaceutically acceptable salt thereof, prepared without a granulation step, wherein the measured atorvastatin potency of said unit dosage form shows a relative standard deviation (RSD) for atorvastatin potency per unit dosage form of not more than about 7.8%, when said unit dosage form is prepared at a rate greater than 10,000 unit dosage forms per hour per single unit dosage form per machine.

A second aspect of the present invention is a unit dosage form comprising atorvastatin or a pharmaceutically acceptable salt thereof, in combination with at least one active drug, prepared without a granulation step, wherein the measured atorvastatin ptoency of said unit dosage form shows a relative standard deviation (RSD) for atorvastatin potency per unit dosage form of not more than about 7.8%, when said unit dosage form is prepared at a rate greater than 10,000 unit dosage forms per hour per single unit dosage form per machine.

A third aspect of the present invention is a method for preparing tablets or capsules of atorvastatin or a pharmaceutically acceptable salt thereof comprising the following steps:

(a) preparing an atorvastatin composition by blending atorvastatin or a pharmaceutically acceptable salt thereof, and one or more excipients suitable for use without a granulation step in a mixer; and

(b) filling a tablet die or capsule and compressing or sealing such that the measured atorvastatin potency shows a relative standard deviation (RSD) for atorvastatin activity per tablet or capsule of not more than about 7.8% when said tablets or capsules are prepared on a tablet press or capsule filler such that greater than 10,000 tablets or capsules are produced per hour per machine.

A fourth aspect of the present invention is a method for preparing tablets or capsules of atorvastatin or a pharmaceutically acceptable salt thereof in combination with at least one active drug comprising the following steps:

(a) preparing an atorvastatin composition by blending atorvastatin or a pharmaceutically acceptable salt thereof and at least one active drug, and one or more excipients suitable for use without a granulation step in a mixer; and

(b) filling a tablet die or capsule and compressing or sealing such that the measured atorvastatin potency shows a relative standard deviation (RSD) for atorvastatin activity per tablet or capsule of not more than about 7.8% when said tablets or capsules are prepared on a tablet press or capsule filler such that greater than 10,000 tablets or capsules are produced per hour per machine.

A fifth aspect of the present invention is a therapeutic package or kit suitable for commercial sale, comprising a container and a therapeutically effective amount of unit dosage forms of atorvastatin or a pharmaceutically acceptable salt thereof prepared without a granulation.

A sixth aspect of the present invention is a method of using the pharmaceutical unit dosage forms to treat subjects suffering from hypercholesterolemia and/or hyperlipidemia, osteoporosis, benign prostatic hyperplasia (BPH), and Alzheimer’s disease.

BRIEF DESCRIPTION OF THE DRAWINGS

This invention is further described by the following non-limiting examples, which refer to the accompanying Figure 1, short particulars of which are given below.

Figure 1

Figure 1 shows the correlation between the percent relative standard deviation (%RSD) in potency for unit dosage forms prepared using a combination of atorvastatin, magnesium stearate and various diluents, and the segregation numbers for these blends.

DETAILED DESCRIPTION OF THE INVENTION

Atorvastatin can readily be prepared as described in United States Patent Numbers 5,273,995 and 5,969,156, which are incorporated herein by reference. The hemicalcium salt of atorvastatin is currently sold as Lipitor®.

Atorvastatin exists in a number of morphological forms ranging from highly crystalline forms to forms with varying degrees of disorder. Some of these disordered forms still possess some structure as indicated by powder x-ray diffraction patterns. For the purpose of the present invention, all forms of atorvastatin benefit from the invention and are included within the scope of the invention. Partially or completely disordered forms of atorvastatin particularly benefit from the invention. Partially or completely disordered forms of atorvastatin that are amorphous or predominantly amorphous derive the greatest benefit from the present invention. Such forms can be prepared, for example, from crystalline atorvastatin using procedures disclosed in United States Patent Number 6,087,511, which is incorporated herein by reference. Alternatively, amorphous material can be prepared according to the processes disclosed in US-A-2005/0032880. For the practice of the present invention, non-crystalline and crystalline atorvastatin can be prepared by any method known in the art. Preferred forms of atorvastatin are described in United States Patents 5,969,156, 6,121,461 and 6,605,729; and in International Patent Applications WO 01/36384, WO 02/41834, WO 02/43732, WO 02/051804, WO 02/057229, WO 3/011826, WO 03/050085, WO 03/070702, and WO 04/022053, which are incorporated herein by reference.

The atorvastatin can be used in the form in which it is prepared, or it can be subjected to a process which changes the physical nature of the particles. For example, the material can be milled by any process known in the art. Non-exclusive examples of such processes include mechanical milling and jet milling. The particles produced either directly from the process of forming amorphous atorvastatin or after a milling operation preferably provide average particle diameters in the range of 1-100 μm.

Pharmaceutically acceptable base addition salts of atorvastatin are formed with metals or amines, such as alkaline and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine (see, for example, Berge, S.M., et al., "Pharmaceutical Salts", J. Pharm. Sci., 1977; 66:1).

The base addition salts of atorvastatin are prepared by contacting the free acid form with a sufficient amount of the desired base to produce the salt in the conventional manner. Additionally, atorvastatin can exist in unsolvated forms as well as solvated forms, including hydrated forms. The scope of the present invention encompasses such salt and solvated forms of atorvastatin.

Forms of atorvastatin that are at least somewhat disordered or a mixture of crystalline and disordered forms of atorvastatin benefit most significantly from the present invention. By somewhat disordered, it is meant that the line width (peak width at half the height of the peak) of any of the peaks measured using powder x-ray diffraction (PXRD) have 2 theta values greater than about 2°. Amorphous or predominantly amorphous forms of atorvastatin, which especially benefit from the present invention, are characterized by having very broad, featureless peaks. It should be noted that combinations of crystalline and at least somewhat disordered forms of atorvastatin would show both sharp (i.e., less than 2° values for 2 theta) and broad peaks (i.e., greater than 2°), and such combinations of forms benefit from the present invention.

Atorvastatin has been found to be an effective drug even at relatively low doses. In fact, by keeping the dose low for a given patient, it is possible to minimize side-effects while still maintaining drug efficacy. It is therefore desirable to provide atorvastatin in a form capable of providing a low dose to the patient. For the purposes of the present invention, the dose provided by the final dosage form of atorvastatin is preferably between 0.5 and 120 mgA (where mgA means milligrams of active drug based on the free acid); more preferably, between 5 and 80 mgA.

For convenience and ease of patient compliance, most drugs are delivered in the form of unit dosage forms. For solid drug substances, these unit dosage forms are generally in the form of tablets, capsules, sachets, chewable tablets and fast dissolving dosage forms. In the present invention, the dosage form is preferably in the form of a capsule or tablet; most preferably in the form of a tablet. The preparation of these forms involves a necessary step of some type of powder filling, either by volume or weight. For example, in production of tablets and capsules, powder is volume filled into a die or capsule, respectively. In order for the unit dosage forms to have the same potency (i.e., amount of drug per unit dosage form) within allowable margins (relative standard deviation, RSD, of less than 6% to meet Stage I, and less than 7.8% to meet Stage II of the United States Pharmacopoeia, USP, guidelines), there must not be any significant

segregation of the active drug from the excipients. This is especially significant for highly dilute forms. The present invention discloses compositions that provide reproducible potency for a fixed weight of active atorvastatin plus excipients without the need for granulation. Moreover, this potency control is maintained through the process of producing product. Such compositions provide atorvastatin with potency (mgA per gram of blend) variability of less than an RSD of 7.8%; more preferably, less than 6.0%. In addition, the present compositions provide for good powder flow such that weight control is maintained between unit dosage forms produced with such compositions. Preferably, such compositions provide unit dosage forms with weight control within an RSD of 6%; more preferably, within 5%; most preferably, within 4%. Combining the weight control and the potency control allows the present formulations to provide unit dosage forms with actual potency of atorvastatin per unit dosage form having an RSD preferably less than 7.8%; more preferably less than 6.0%. That these standards are met without granulation provides for a more efficient manufacturing process by reducing the number of unit operations as compared to processes involving granulations. In production of unit dosage forms of atorvastatin without a granulation step, it is possible to produce such unit dosage forms without the present compositions using processes unsuitable to commercial production. For example, individual ingredients could be weighed into a capsule directly. The present invention, therefore, is preferably used in conjunction with high-speed production equipment. More specifically, preferred formulations allow potency control of unit dosage forms to less than 7.8% RSD (more preferably, less than 6.0% RSD) when used with a single unit dosage form production equipment at a rate of greater than 10,000 unit dosage forms per hour; more preferably, greater than 25,000 unit dosage forms per hour; still more preferably, greater than 50,000 unit dosage forms per hour. Preferred unit dosage production equipment or machines include rotary tablet presses and commercial capsule filling equipment. Non-exclusive examples of commercial rotary tablet presses include those produced by Niro Pharma Systems (Columbia, MD), Kilian and Company (Horsham, PA), Korsch (Berline, Germany) and Elizabet-Hata International (North Huntingdon, PA). Non-exclusive examples of commercial capsule filling equipment include those made by Capsugel (Morris Plains, NJ) and CapPlus Technologies (Phoenix, AZ).

Measurement of the potency of unit dosage forms of atorvastatin is necessary in determining the variability in activity between unit dosage forms. An extraction process against a standard with independently known drug levels best determines such potency. The potency analysis is best conducted using reverse phase high performance liquid chromatography (HPLC) techniques such as those known in the art relative to standards. RSD measurements, for the purpose of the present invention, are best carried out using sampling during a process for forming the unit dosage form. More specifically, unit dosage forms can be sampled from a preparation process at various time points (beginning, middle and end of the run). In determining an RSD value, at least three unit dosage forms should be measured from each section. An alternative analytical technique for determining the potency of a sample of drug involves the use of ultraviolet-visible absorption spectroscopy. In this technique, the absorbance corresponding to atorvastatin is used to quantify the concentration of atorvastatin in a sample (taking care that no excipient has interfering absorptions), as is known in the art.

The present invention discloses processes and formulations that provide atorvastatin in a pure and stable form. The term "impurities" describes materials in the drug substance present from the synthesis and purification process and any drug-based materials formed in the preparation of the unit dosage form. The term "degradants" refers to any drug-based materials generated after the preparation of the unit dosage form. Analysis of impurities and degradants is done using reverse phase HPLC techniques on extracted samples as is known in the art. Calculations of the amount of impurities and degradants are expressed as the integrated area percent of the degradant or impurity peak(s) divided by the integrated area percent of all drug related peaks.

In the formulation of atorvastatin without granulation, combinations of diluents, binders, disintegrants, flavorants and lubricants are used to provide the properties needed for the unit dosage form as is known in the art. For example, for preparation of tablets, the combination provides for adequate tablet hardness upon compression while providing rapid disintegration *in vivo*. Although there is a wide degree of latitude in formulating atorvastatin to meet these conditions, typically such compositions contain about 1-40% weight:weight (w:w) drug, about 5-10% disintegrant, about 0-10% binder and about 0.5-2% lubricant, with the remaining percentage comprising the present inventive diluents. Preferred disintegrants include carboxymethylcellulose, hydroxyproyl cellulose (low-substituted), microcrystalline cellulose, powdered cellulose, colloidal silicon dioxide, croscarmellose sodium, crospovidone, magnesium aluminum silicate, methylcellulose, polacrilin potassium, povidone, sodium alginate, sodium starch glycolate and starches. Preferred binders include acacia, carboxymethylcellulose, hydroxyethylcellulose, hydroxypropylcellulose, dextrin, gelatin, guar gum, hydroxypropyl methylcellulose, magnesium aluminum silicate, maltodextrin, methylcellulose, polyethylene oxide, polymethacrylates, povidone, sodium alginate, starches and zein. Preferred lubricants include calcium stearate, glyceryl palmitostearate, magnesium oxide, poloxamer, polyethylene glycol, polyvinyl alcohol, sodium benzoate, sodium lauryl sulfate, sodium stearyl fumarate, stearic acid, talc, zinc stearate and magnesium stearate.

In providing atorvastatin formulations in forms suitable for unit dosage formation without granulation, the drug and excipients are typically mixed as powders. This mixing can be carried out using any of the mixing techniques known in the art. The mixing is preferably carried out using a high shear mixer, V-blender (or other twin-shell blender), bin blender or Turbula™ mixer-shaker. Blending is typically carried out first without the addition of a lubricant for sufficient

time to assure complete mixing. At that point, the lubricant is typically added followed by a short (about 1-10 minute) further mixing period. Once the blend is made, unit dosage forms are prepared by procedures known in the art. Preferably, unit dosage forms are made on rotary tablet presses or capsule filling machines. The dosage forms thus prepared can then optionally be coated with a film designed to provide ease of swallowing, a proprietary or identification appearance and/or protection of the dosage form. The final dosage form is then packaged using procedures known in the art. For the present invention, the packaging is preferably in the form of foil-foil cold form blisters, plastic blisters or sealed bottles containing desiccants. Optionally, the packaging can contain active oxygen absorbing materials as is disclosed in EP1243524A2 and EP1241110A1, which are incorporated herein by reference.

Unit dosage forms of atorvastatin that are formed without a granulation step with preferred excipients show low levels of drug-related impurities and degradants. Surprisingly, this low level of impurities and degradants was found even in the absence of added alkalizing agents or alkaline earth metal salts. Even more surprisingly, this low level of impurities and degradants was maintained even when the atorvastatin used was an at least somewhat disordered form of the drug. In particular, it was found that while wet granulated control unit dosage forms of atorvastatin show high levels of drug degradation, unit dosage forms prepared without granulation have greater stability. Those unit dosage forms of atorvastatin prepared without granulation are preferred that contain not more than about 2% total drug related impurities and/or degradants based on the area percent of the impurities/degradants relative to the integrated area of all drug related peaks as determined by HPLC; more preferably, they contain less than 1%; still more preferably, less than 0.7%. In addition, unit dosage forms of atorvastatin prepared without granulation are preferred that provide stability such that upon storage at 40°C and 75% relative humidity (RH) for four weeks, the unit dosage forms contain not more than about 2% total drug related impurities and/or degradants based on the area percent of the impurities/degradants relative to the integrated area of all drug related peaks as determined by HPLC; more preferably, they contain less than 1%; still more preferably, less than 0.7%.

Atorvastatin undergoes two major degradation pathways: lactonization and oxidation. The lactone is formed by internal condensation (loss of water) of the alcohol and carboxylic acid to form a six-membered ring. This is the major degradant of amorphous atorvastatin found upon wet granulation and tablet formation as described in United States Patent Numbers 5,686,104 and 6,126,971, especially in the absence of alkaline earth metal salt additives. We have found, unexpectedly, that the level of the lactone in unit dosage forms, both initially and upon storage under accelerated aging conditions of increased temperature and humidity, can be significantly reduced by combination of the present excipients and production of unit dosage forms without a granulation process. As such, unit dosage forms of atorvastatin prepared without a granulation step are preferably those for which the level of atorvastatin lactone is less than 2% (based on the ratio of lactone peak integration compared to the total drug related peak integrated areas using HPLC) after said unit dosage forms are produced and stored at 40°C/75% RH (where RH represents relative humidity) for four weeks; more preferably, less than 1%.

To minimize bioavailability issues and potential interactions with other drugs in combination dosage forms, in the practice of the present invention, the level of alkaline earth metal salts in the formulation is preferably about 0-5% (w:w); more preferably, about 0-2%; most preferably about 0-1%. It is also preferred that the level of other alkalizing agents in the formulation be about 0-5% (w:w); more preferably, about 0-3%; most preferably about 0-2%. It is also preferred that levels of polymeric amides or polymeric amines be less than about 0-5% (w:w); more preferably, about 0-3%; most preferably about 0-2% of the formulation. Examples of such polymers are disclosed in International Patent Application WO 01/76566A1.

Alkalizing agents are additives or excipients that have the property of increasing the pH of a formulation, when such formulations are added to water. Examples of alkalizing agents include inorganic and organic bases (buffers). Examples of inorganic alkalizing agents include sodium or potassium citrate, carbonate, bicarbonate, phosphate, sulfate, benzoate and ascorbate, and calcium carbonate and magnesium carbonate. The latter two also represent examples of alkaline earth metal salts. Examples of organic alkalizing agents include amines. Specific examples of amines include N-methylglucamine, guanine and arginine.

One of the common mechanisms for segregation of particles is a process of "sifting." Sifting is the process by which small particles move through a matrix of larger ones, or vice versa. This form of segregation will occur if the difference in particle sizes is sufficient (as little as 30% size difference can be associated with sifting), the materials are not bound to each other and there is a mechanism for interparticle motion. In the formation of dosage forms, the interparticle motion can occur during powder mixing or flowing. Using atorvastatin and excipients with similar particle sizes would potentially prevent segregation between the materials during production of unit dosage forms; however, this can be problematic for a number of reasons. As discussed below, to provide adequate flow of powder, mean excipient particle sizes preferably are between about 80-360 μm in diameter. In contrast, the mean atorvastatin particle size is preferably between about 1-100 μm, in diameter. This size is preferred for ease of manufacture, reproducibility in drug dissolution (bioavailability), and to improve potency variability in the dosage form produced with these particles due to statistics of the number of particles in the unit dosage form. The general contrast between the atorvastatin and predominant excipients in particle sizes makes sifting problematic for atorvastatin in the presence of excipients suitable for use without granulation.

A second method of segregation is fluidization. Fluidization results from the tendency of smaller particles to retain air longer than larger particles during processes that involve high rates of powder transfers or mixing. Segregation due to fluidization is especially problematic for particles of less than about 100 μm in size, as is desirable with atorvastatin.

Segregation of small drug particles from larger excipient particles will be minimal if there is a sufficient adhesion of the drug particles to some of the excipient particles. Such adhesion must be sufficient to withstand the shear forces the powder is subject to. We have found that by use of a simple test procedure on binary mixtures of the drug and a single excipient (with a constant low level of magnesium stearate lubricant), excipients having sufficient adhesion to the drug to prevent segregation during unit dosage form preparation (as exemplified by tablet production) can be identified.

In this procedure, a blend of atorvastatin, magnesium stearate (1 wt%) and the test excipient are first blended together using a V-blender or Turbula™ mixer-shaker. The relative weights of the drug and excipient are the same as that to be used in the final proposed composition. The blend is then subjected to a fluidization segregation test using procedures as described in ASTM D6941-03. For example, a fluidization tester, model 6274.01-1, available from Jenike & Johanson Inc. (Westford, MA) can be used. The blend is added to the instrument. Gas is flowed through the powder bed of the instrument separating materials vertically in the column. The instrument allows sampling from various heights of the column. HPLC or ultra-violet/visible absorption analysis of the potency of samples from the top, middle and bottom of the column provides an indication of whether the drug adheres to particular excipients. A segregation number can be defined as the difference in potencies between the top ($P_{top}$) and bottom ($P_{bottom}$) of the fluidized bed, divided by the average potency of the top ($P_{top}$), middle ($P_{middle}$) and bottom ($P_{bottom}$) samples. This can be expressed as:

$$\text{Segregation Number} = [P_{top} - P_{bottom}]/[(P_{top} + P_{middle} + P_{bottom})/3]$$

We have determined an empirical correlation between the variability (RSD) in potency during high-speed production of unit dosage forms (tablets) with the segregation number as shown in Figure 1. Based on this, acceptable uniformity is predicted for a final unit dosage form manufacturing run if the component excipients in combination with the atorvastatin show a segregation number of less than 0.7; more preferably, less than 0.6; and still more preferably, less than 0.5.

Moreover, individual excipients can be characterized with atorvastatin in binary tests. In this case, a standard atorvastatin concentration of 1 wt% can be used (98 wt% excipient and 1 wt% magnesium stearate). As above, a segregation number can be calculated from fluidization segregation testing by taking the ratio of the difference in top and bottom sample potency to the overall average potency of the top, middle and bottom samples. The final suitability of excipient blends in use with atorvastatin depends on a weighted average of the excipients' respective segregation numbers. For example, for a 60:40 weight ratio of two materials with individual segregation numbers (with a particular form of atorvastatin) of 0.3 and 0.6, respectively, the weighted average segregation number would be [0.60 X 0.3 + 0.40 X 0.6] = 0.42. Preferred compositions show weighted average segregation numbers less than 0.7 based on binary values with atorvastatin; more preferably, less than 0.6; and still more preferably, less than 0.5.

Preferred excipients are diluents, which preferably comprise greater than or equal to 50 wt% of the excipients in the composition with atorvastatin. Preferred diluents for preparation of atorvastatin unit dosage forms without a granulation step provide weighted average segregation numbers less than 0.7; more preferably, less than 0.6; and still more preferably less than 0.5. Potential diluents are identified as such in "Handbook of Pharmaceutical Excipients, 3rd Edition" (A. H. Kibbe, Editor; Pharmaceutical Press, London; 2000). These include the following non-limiting examples: calcium phosphate, calcium sulfate, carboxymethylcellulose calcium, cellulose, cellulose acetate, dextrates, dextrin, dextrose, fructose, glyceryl palmitostearate, hydrogenated vegetable oil, kaolin, lactitol, lactose, magnesium carbonate, magnesium oxide, maltitol, maltodextrin, maltose, polymethacrylates, pregelatinized starch, silicified microcrystalline cellulose, sodium chloride, sorbitol, starch, sucrose and talc.

In testing the excipients, it is important that the particular form and particle size of atorvastatin be used that is desired for the final dosage form. It has been found that the method of preparation of atorvastatin affects the segregation number and corresponding content uniformity of unit dosage forms produced therein. For example, amorphous atorvastatin produced by spray drying gives different values than amorphous atorvastatin produced by precipitation.

For diluents to be useful in commercial production of atorvastatin unit dosage forms, flow of the diluents must be adequate to assure weight control. As such, we have found that greater than about 50% by weight of the diluents used in production of atorvastatin unit dosage forms preferably have mean particles sizes of between about 80 and 360 μm in diameter; more preferably, between 90 and 280 μm; even more preferably, more than 70% of the diluents used in the production of atorvastatin unit dosage forms have mean particles sizes of between about 80 and 360 μm in diameter. Mean particle sizes can be measured using a laser diffraction particle size instrument such as those made by Sympatec GmbH (Goslar, Germany). Mean particle sizes, for the purpose of the present invention, can be considered the size for which 50% of the particles have diameters smaller than the indicated number. Alternatively, particle size can

be assessed using sieve analysis. In this case, the sieve size that retains half the weight of material (half passes through) corresponds to the mean particle size. Preferred diluents have mean particle sizes based on sieve analysis such that 50 weight % passes through sieves between a number 200 (ASTM) (corresponding to 75 μm) and a number 45 (corresponding to 355 μm); more preferably, between a number 170 (90 μm) and number 50 (300 μm).

Coupling the segregation tendency of diluents with atorvastatin and the particle sizes of said diluents, defines a list of preferred diluents for formulation of atorvastatin. As such, preferred diluents for formulation of atorvastatin without a granulation step include large particle sizes of lactose monohydrate (for example, Fast Flo 316™, available from Foremost Farms, Baraboo, WI, which has a mean particle size of 101 μm), large particle size lactose anhydrous (for example, the grade of lactose from Quest International (Sheffield Products), Hoffman Estates, IL, which has a mean particle size of 136 μm), large particle size microcrystalline cellulose (for example, Avicel PH200™, available from FMC Biopolymers, Philadelphia, PA, which has a mean particle size of 180 μm) and sodium chloride (for example, the granular grade available from Mallinckrodt Baker, Inc., Phillipsburg, NJ, which has a mean particle size of 355 μm). Examples of excipients commonly used in pharmaceutical formulations which are not preferred excipients in the present invention include small particle size microcrystalline cellulose (for example, Avicel PH105™, which has a mean particle size of 20 μm, and Avicel PH103, 113 and 301, each with a mean particle size of 50 μm) and small particle size lactose (for example the following lactose grades which all have mean particle sizes below 75 μm: Pharmatose™ 125M, 150M, 200M, 350M, 450M, available from DMV International, Vegnel, The Netherlands; Impalpable™ #312 and #313, available from Foremost Ingredients Group, Rothschild, WI; and monohydrate 80M, monohydrate impalpable and anhydrous impalpable, available from Quest International, Sheffield Products, Hoffman Estates, IL).

The present invention provides for compositions of atorvastatin which are particularly well suited for combination products with other drug substances because of the lower tendency for atorvastatin to segregate with the diluents of the present invention. Non-limiting examples of drugs which may benefit from combinations with the inventive atorvastatin compositions and processes include torcetrapib and amlodipine and pharmaceutically acceptable salts thereof.

Compositions of atorvastatin according to the present invention can be combined with a least one other active drug to form unit dosage forms. Preferred unit dosage forms include tablets and capsules. In the combination of the atorvastatin composition with at least one other active drug to form a unit dosage form, the following non-limiting list describes options for such unit dosage forms: (a) a blend of the atorvastatin, excipients and the other active drug formed into unit dosage forms; (b) a blend of atorvastatin, excipients and a granulation of the other drug, optionally with excipients, formed into unit dosage forms; and (c) a bilayer tablet comprising atorvastatin with excipients in one layer and the other drug and optional excipients in the other layer.

The present invention relates to the treatment of diseases and conditions in a subject, such as, hyperlipidemia and/or hypercholesterolemia, osteoporosis, benign prostatic hyperplasia (BPH), and Alzheimer's disease with atorvastatin or a pharmaceutically acceptable salt thereof as described above that may be administered in a unit dosage form having low levels of degradation products and/or impurities contained in a therapeutic package or kit. The kit includes the unit dosage form and a container. Typically, the kit includes directions for administration of the unit dosage form. The container can be in any conventional shape or form as known in the art, for example, a paper box, a glass or plastic bottle, or a blister pack with individual dosage forms pressing out of the back according to a therapeutic schedule.

The following non-limiting examples illustrate the inventors' preferred methods for preparing and using the pharmaceutical compositions of the present invention.

## EXAMPLE 1

### GENERAL METHOD FOR PREPARATION OF SPRAY-DRIED AMORPHOUS ATORVASTATIN

Spray dried amorphous atorvastatin, an example of disordered atorvastatin as previously described in the Detailed Description of the Invention, used in some of the following examples was prepared according to the process described in concurrently filed U.S. Patent Application, commonly owned, attorney case number PC-25825, serial number _____, by first dissolving atorvastatin calcium (U.S. Patent No. 5,273,995) in methanol to make a 5% (w:w) solution. This solution was sprayed into a Niro PSD-1 spray dryer at a rate of 170 gram/minute (g/min) using nitrogen as the atomizing gas. The inlet temperature was 195°C and the outlet temperature was 60°C. After spray drying, the powder was tray-dried in an oven at 40°C for 12 hrs to afford amorphous atorvastatin.

## EXAMPLE 2

### GENERAL METHOD FOR PREPARATION OF PRECIPITATED AMORPHOUS ATORVASTATIN

Precipitated amorphous atorvastatin, an example of disordered atorvastatin as described in the Detailed Description of the Invention, used in some of the following examples was prepared according to the process described in

concurrently filed U.S. Patent Application, commonly attorney case number PC32139, serial number _____ by first dissolving 1.80 kg of atorvastatin calcium (U.S. Patent No. 5,273,995) in 18 L of tetrahydrofuran (THF) by stirring in a jacketed glass reactor with overhead stirring. The THF solution was added over a two-hour period to a mixture containing heptane (55 L) and 2-propanol (1.125 L) in a jacketed reactor using constant agitation by an overhead stirrer while maintaining a temperature between 15-25°C. The resulting slurry was stirred for one hour then slowly cooled to 0-5°C over a one-hour period. The precipitated material was isolated on a horizontal plate filter covered with polyethylene cloth by vacuum filtration and dried under vacuum (20-30 inches pressure) at 50-60°C to yield a total of 1.6 kg of amorphous atorvastatin.

## EXAMPLE 3

### PREPARATION OF AMORPHOUS ATORVASTATIN TABLETS USING A WET GRANULATION

The following materials were added to a 950-cc amber bottle: 2.59 g of spray dried amorphous atorvastatin (prepared as described in Example 1), 78.00 g of microcrystalline cellulose (Avicel™ PH102, FMC Biopolymer, Philadelphia, PA), 101.41 g of lactose (hydrous, Foremost Farms USA, Rothschild, WI), 6.00 g of croscarmellose sodium (Ac-Di-Sol™ FMC Biopolymer, Philadelphia, PA), and 4.000 g of hydroxypropyl cellulose (Klucel ™ EXF, Hercules Incorporated, Aqualon Division, Wilmington, DE). The materials were bottle blended for 10 minutes (min.) using a Turbula™ mixer (Turbula Shaker Mixer, Willy A. Bachofen AG Maschinenfabrik, Basel, Switzerland) and then discharged and sieved through a 30 mesh screen to delump. The material was then put back into the bottle and Turbula™ mixed an additional 10 minutes. The bottle-blended material was added to a Pro-Cept Mi-Mi Pro high shear wet granulator (Pro-Cept n.v., B- 9060 Zelzate, Belgium) using a 1.7 L bowl. The materials were dry mixed for two minutes at a chopper speed of 1000 revolutions per minute (rpm) and an impeller speed of 400 rpm, then the impeller speed was increased to 600 rpm maintaining the chopper speed. At this point, 90 mL of water was added at a rate of 30 mL/min. in three separate additions (60 mL, 15mL, 15 mL) over a total of 5.5 min. wet mixing. The material was discharged and wet sieved by hand through a #10 mesh sieve. The sieved material was dried by placing on a polyethylene lined tray in a Gruenberg™ forced hot air oven (Gruenberg Oven Co., Williamsport, PA) at 50°C for 16 hrs. The dried material was then milled using a Fitzpatrick L1A mill (The Fitzpatrick Co., Elmhurst, IL) with a 0.040" Conidur rasping screen at 500 rpm. To 175.0 g of the blend was added 5.469 g of Ac-Di-Sol™ and the mixture was bottle blended (950-cc amber bottle) using a Turbula™ mixer for 5 min. Magnesium stearate (Mallinckrodt Inc., St. Louis, MO) 1.822 g was then added and the mixture Turbula™ blended an additional 3 min. to complete the formulation. Tablets (~250) were prepared using an F-press (Manesty F-Press, Liverpool, United Kingdom) with 13/32" standard round concave (SRC) tooling, with a target weight of 450 mg (+/- 3%) and a target hardness of 12 kP (range 10-14kp). A total of 12 tablets were placed in 30-cc high density polyethylene (HDPE) bottles sealed using heat induction seal (HIS) closures, sealed using a heat induction sealer (Enercon Industries Corp., Menomonee, WI). Samples were stored for 4 weeks at 40°C and 75% relative humidity (RH). Samples were analyzed for the level of atorvastatin lactone by adding one tablet to 50 mL of 1:1 (v:v) of a 0.05M ammonium citrate buffer (pH 7.4):acetonitrile and shaking for 20 min. The material was then filtered using a Gelman Acrodisc polytetrafluoroethylene membrane (0.45 μm pore size), and analyzed using high-pressure liquid chromatography (HPLC) (Phenomenex, Ultremex C18 column, 25.0 cm X 4.6 mm, HPLC HP 1100 series, Agilent Corp., Wilmington, DE, 20 μl injection volume, flow of 1.5 mL/min; mobile phase of 53:27:20 (v:v:v) 0.05M ammonium citrate (pH 4.0): acetonitrile:tetrahydrofuran; detection at 244 nm). The lactone level was found to be 25.4% (based on a ratio of the lactone peak to the total peak areas of all peaks).

## EXAMPLE 4

### PREPARATION OF AMORPHOUS ATORVASTATIN TABLETS BY DIRECT COMPRESSION

The following materials were added to a 950-cc amber glass bottle: 2.59 g of atorvastatin (prepared as described in Example 1), 78.00 g microcrystalline cellulose (Avicel PH102™; FMC Corp., Philadelphia, PA), 101.41 g lactose, hydrous (REG 310; Foremost Farms USA, Rothschild, WI), 4.00 g hydroxypropyl cellulose (Klucel EXF™; Aqualon, Wilmington, DE), and 12.00 g croscarmellose sodium (Ac-Di-Sol™; FMC Corp., Philadelphia, PA). The combination of the above ingredients was mixed using a Turbula™ mixer-shaker (Glen Mills, Clifton, NJ) for 10 min. The blend was then passed through a stainless steel sieve (#30 mesh) to delump, after which an additional 10 min. of mixing was performed. Lastly, 2.00 g magnesium stearate (Mallinckrodt Co., St. Louis, MO) was added to the amber glass bottle and the contents blended using the Turbula™ for 3 min. Tablets were made using a single station Manesty F-Press (Manesty, Liverpool, United Kingdom). A 13/32" standard round concave (SRC) punch and die was used to produce tablets with weights of 450 mg each. The average tablet hardness was 11 kP with a range of 9-14 kP (tablet hardness was tested using a Schleuniger Tablet Hardness Tester, Dr. Schleuniger Pharmatron AG, Solothurn, Switzerland). The average tablet

weight was 451.2 mg with an RSD of 1.3 %. Tablets were packaged and studied for stability (4 weeks; 40°C/75% RH) as described in Example 3. The level of lactone found under these conditions was 0.12% (based on a ratio of the lactone peak to the total peak areas of all peaks).

Comparison between Examples 3 and 4 show the unexpected advantage of the direct compression process for producing a unit dosage form of atorvastatin with higher purity than observed with wet granulation.

## EXAMPLE 5

### PREPARATION OF BLENDS OF SPRAY-DRIED ATORVASTATIN AND DILUENTS AND ANALYSIS OF SEGREGATION

In each of the following blends, spray-dried amorphous atorvastatin (prepared as described in Example 1) was used. The segregation numbers for these binary blends of atorvastatin were determined using a fluidization segregation tester (model 6274.01-1; Jenike & Johanson, Westford, MA). The fluidization chamber was filled with approximately 75-cc of blend to be tested. The fixed test parameters were as follows: air pressure, 25 psi; hold time, 120 sec.; ramp time, 30 sec. Airflow settings to fluidize each blend are inherent to each formulation and indicated below. The three fluidized column sections (top, middle, and bottom) were each riffled (Model RR-5 Rotary Micro Riffler, Quantachrome Instruments, Boynton Beach, FL) to obtain representative samples for analytical analysis. The results in all cases are reported in Table 1. In Table 1 are presented the observed potencies as percent of intended potencies (% Intent).

(a) A 4-quart V-blender (Patterson-Kelly Corp., East Stroudsburg, PA) was charged with 1959.0 g of delumped (20 mesh screen sieved) calcium phosphate dibasic anhydrous (A-tab™, Rhodia Corp., Etoile Part-Dieu, France). On top of this was placed 21.1 g of atorvastatin (from Example 1), which was spread onto the diluent bed using a spatula. The combination was blended for 15 min. To the blender was added 20.0 g of magnesium stearate (Mallinckrodt Corp., Hazelwood, MO), and the mixture was blended for 5 min. Segregation analysis was conducted with air flow settings of 10 and 15, for low and high, respectively. The potency values were determined for top, middle and bottom sections by weighing 2.2 g of blend and adding 250 mL of 1:1 (v:v) 0.05M ammonium citrate buffer (pH 7.4):acetonitrile and shaking for 20 min. The material was then filtered using a Gelman Acrodisc polytetrafluroroethylene membrane (0.45μm pore size), and analyzed using HPLC (Phenomenex, Ultremex C18 column, 25.0 cm X 4.6 mm, HPLC HP 1100 series, 20 μL injection volume, flow of 1.5 mL/min; mobile phase of 53:27:20 (v:v:v) 0.05M ammonium citrate (pH4.0):acetonitrileaetrahydrofuran; detection at 244 nm

(b) A 4-quart V-blender (Patterson-Kelly Corp., East Stroudsburg, PA) was charged with 1959.0 g of delumped (20 mesh screen sieved) lactose anhydrous (direct tableting grade, Quest International (Sheffield Products), Hoffman Estates, IL). On top of this was placed 21.1 g of atorvastatin (from Example 1), which was spread onto the diluent bed using a spatula. The combination was blended for 15 min. To the blender was added 19.9 g of magnesium stearate (Mallinckrodt Corp., Hazelwood, MO), and the mixture was blended for 5 min. Segregation analysis was conducted with air flow settings of 10 and 15, for low and high, respectively. The potency values were determined for top, middle and bottom sections in the same manner as in Example 5a with a sample of 2.0 g.

(c) A 4-quart V-blender (Patterson-Kelly Corp., East Stroudsburg, PA) was charged with 1077.4 g of lactose monohydrate (REG 310™, Foremost Farms, Baraboo, WI). On top of this was placed 11.6 g of atorvastatin (from Example 1), which was spread onto the diluent bed using a spatula. The combination was blended for 15 min. To the blender was added 11.0 g of magnesium stearate (Mallinckrodt Corp., Hazelwood, MO), and the mixture was blended for 5 min. Segregation analysis was conducted with air flow settings of 15 and 20, for low and high, respectively. The potency values were determined for top, middle and bottom sections in the same manner as described in Example 5a with top sample using 1.4 g, and the middle and bottom samples using 2.0 g (extracted into 100 mL of extraction solvent).

(d) A 4-quart V-blender (Patterson-Kelly Corp., East Stroudsburg, PA) was charged with 1077.1 g of lactose monohydrate spray dried (Fast Flo 316™, Foremost Farms, Baraboo, WI). On top of this was placed 11.6 g of atorvastatin (from Example 1), which was spread onto the diluent bed using a spatula. The combination was blended for 15 min. To the blender was added 11.0 g of magnesium stearate (Mallinckrodt Corp., Hazelwood, MO), and the mixture was blended for 5 min. Segregation analysis was conducted with air flow settings of 20 and 30, for low and high, respectively. The potency values were determined as described in Example 5a with a sample size of 2.0 g and extraction volume of 100 mL.

(e) A 4-quart V-blender (Patterson-Kelly Corp., East Stroudsburg, PA) was charged with 881.5 g of microcrystalline cellulose (Avicel PH200™, FMC Biopolymers, Philadelphia, PA). On top of this was placed 9.6 g of atorvastatin (from Example 1), which was spread onto the diluent bed using a spatula. The combination was blended for 15 min. To the blender was added 9.0 g of magnesium stearate (Mallinckrodt Corp., Hazelwood, MO), and the mixture was blended for 5 min. Segregation analysis was conducted with air flow settings of 4 and 10, for low and high, respectively.

The potency values were determined for top, middle and bottom sections as described in Example 5a with a sample size of 1.3 g and the extraction volume of 100 mL.

(f) A 4-quart V-blender (Patterson-Kelly Corp., East Stroudsburg, PA) was charged with 1959.0 g of delumped (30 mesh screen sieved) sucrose direct tableting (DI-PAC™, Domino Foods, Inc., Domino Specialty Ingredients, Baltimore, MD). On top of this was placed 21.1 g of atorvastatin (from Example 1), which was spread onto the diluent bed using a spatula. The combination was blended for 15 min. To the blender was added 20.0 g of magnesium stearate (Mallinckrodt Corp., Hazelwood, MO), and the mixture was blended for 5 min. Segregation analysis was conducted with air flow settings of 18 and 25, for low and high, respectively. The potency values were determined for top, middle and bottom sections as described in Example 5a with a sample size of 2.3 g.

(g) A 2-quart V-blender (Patterson-Kelly Corp., East Stroudsburg, PA) was charged with 1467.0 g of delumped (14 mesh screen sieved) sodium chloride (granular grade, Mallinckrodt Baker, Inc., Phillipsburg, NJ). On top of this was placed 15.8 g of atorvastatin (from Example 1), which was spread onto the diluent bed using a spatula. The combination was blended for 15 min. To the blender was added 14.9 g of magnesium stearate (Mallinckrodt Corp., Hazelwood, MO), and the mixture was blended for 5 min. Segregation analysis was conducted with air flow settings of 50 and 80, for low and high, respectively. The potency values were determined for top, middle and bottom sections as described in Example 5a with a sample size of 4.5 g and an extraction volume of 500 mL.

(h) Into a 950-cc amber glass bottle was added in order: 97.97 g of spray dried lactose monohydrate (Foremost Farms USA, Rothschild, WI), 2.12 g of spray-dried amorphous atorvastatin (from Example 1), and 97.94 g of spray dried lactose monohydrate. The ingredients were blended 10 min. using a Turbula™ shaker-mixer (Willy A. Bachofen AG Maschinenfabrak, Basel, Switzerland). The blend was delumped by passing it through a stainless steel, #30 U.S. Standard sieve. Then, the blend was mixed 10 min. using the Turbula™. Lastly, 1.99 g of vegetable sourced, magnesium stearate (Mallinckrodt, St. Louis, MO) was added and the contents blended 3 min. using the Turbula™. Segregation analysis was conducted with air flow settings of 4 and 11, for low and high, respectively. The potency values were determined for top, middle and bottom sections by combining 2.3 g of blend with 250 mL of 1:1 (v:v) deionized water:acetonitrile and shaking for 30 min. The material was then filtered using a Gelman Acrodisc polytetrafluroroethylene membrane (0.45μm pore size), and analyzed using a UV-Vis Spectrophotometer (Model 8453, Agilent Corp., Wilmington, DE, 0.1 cm cell path-length, analysis at 244 nm).

(i) Into a 950-cc amber glass bottle was added in order: 97.94 g of calcium phosphate dibasic dihydrate (Emcompress™, JRS Pharma LP, Chicago Heights, IL), 2.08 g of atorvastatin (from Example 1), and 97.94 g of calcium phosphate dibasic dihydrate (Emcompress™). The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate was added and the contents blended 3 min. using the Turbula™. Segregation analysis was conducted with air flow settings of 8 and 15, for low and high, respectively. The potency values were determined for top, middle and bottom sections as described in Example 5h with a sample size of 3.0 g.

(j) Into a 950-cc amber glass bottle was added in order: 97.94 g of microcrystalline cellulose (Avicel PH 102™, FMC BioPolymers, Newark, DE), 2.08 g of atorvastatin (from Example 1), and 97.94 g of microcrystalline cellulose (Avicel PH 102™). The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate was added and the contents blended 3 min. using the Turbula™. Segregation analysis was conducted with air flow settings of 6 and 15, for low and high, respectively. The potency values were determined for top, middle and bottom sections as described in Example 5h with a sample size of 1.3 g and an extraction volume of 100 mL.

(k) Into a 950-cc amber glass bottle was added in order: 97.96 g of granular mannitol (Mannogem 2080™, SPI Polyols, New Castle, DE), 2.12 g of atorvastatin (from Example 1), and 97.94 g of granular mannitol (Mannogem 2080™). The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate was added and the contents blended three minutes using the Turbula™. Segregation analysis was conducted with air flow settings of 14 and 30, for low and high, respectively. The potency values were determined for top, middle and bottom sections as described in Example 5h with a sample size of 2.0 g and extraction volume of 200 mL.

(l) Into a 950-cc amber glass bottle was added in order: 97.94 g of mannitol powder (EMD Chemicals, Gibbstown, NJ), 2.12 g of atorvastatin (from Example 1), and 97.90 g of mannitol powder. The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate were added and the contents blended three minutes using the Turbula™. Segregation analysis was conducted with air flow settings of 4 and 9, for low and high, respectively. The potency values were determined for top, middle and bottom sections as described in Example 5h with a sample size of 2.0 g and an extraction volume

of 200 mL.

(m) Into a 950-cc amber glass bottle was added in order: 97.94 g of spray-dried mannitol (SPI Polyols, New Castle, DE), 2.12 g of atorvastatin (from Example 1), and 97.92 g of spray-dried mannitol. The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate were added and the contents blended three minutes using the Turbula™. Segregation analysis was conducted with air flow settings of 6 and 12, for low and high, respectively. The potency values were determined for top, middle and bottom sections as described in Example 5h with a sample size of 1.5 g and an extraction volume of 100 mL.

(n) Into a 950-cc amber glass bottle was added in order: 97.94 g of granular xylitol (Xylitol C Granular, Danisco, Thomson, IL), 2.12 g of atorvastatin (from Example 1), and 97.94 g of granular xylitol. The ingredients were blended 10 min. using a Turbula™ shaker-mixer. The blend was delumped by passing it through a stainless steel, #30 U.S. Standard sieve. The formulation was then blended 10 min. using the Turbula™. Lastly, 2.00 g of vegetable sourced, magnesium stearate was added and the contents blended three minutes using the Turbula™. Segregation analysis was conducted with air flow settings of 15 and 29, for low and high, respectively. The potency values were determined for top, middle and bottom sections as described in Example 5h with a sample size of 3.0 g.

Table 1. Results of fluidization testing for binary blends of spray-dried atorvastatin and diluents.

| Example | Top Sample, % Intent | Middle Sample, % Intent | Bottom Sample, % Intent | Segregation Number |
|---|---|---|---|---|
| 5a | 151.9 | 87.9 | 44.0 | 1.14 |
| 5b | 110.8 | 86.7 | 76.2 | 0.38 |
| 5c | 108.8 | 97.8 | 96.8 | 0.12 |
| 5d | 98.1 | 97.7 | 95.1 | 0.03 |
| 5e | 104.8 | 87.6 | 80.9 | 0.26 |
| 5f | 122.2 | 64.4 | 43.1 | 1.03 |
| 5g | 108.1 | 89.9 | 80.4 | 0.30 |
| 5h | 100.9 | 98.0 | 81.6 | 0.21 |
| 5i | 137.4 | 48.7 | 55.8 | 1.01 |
| 5j | 93.9 | 90.3 | 88.7 | 0.06 |
| 5k | 105.7 | 65.4 | 44.5 | 0.85 |
| 5l | 96.0 | 98.3 | 89.8 | 0.07 |
| 5m | 85.9 | 83.9 | 83.8 | 0.02 |
| 5n | 93.0 | 49.0 | 39.5 | 0.88 |

## EXAMPLE 6

### PREPARATION OF BLENDS OF PRECIPITATED ATORVASTATIN AND DILUENTS AND ANALYSIS OF SEGREGATION

Formulations containing precipitated amorphous atorvastatin (prepared as described in Example 2) were combined with one diluent, and magnesium stearate (Mallinckrodt Co., St. Louis, MO) according to the following general process: Into a 950-cc amber glass bottle was added in order, 97.9 g of the diluent indicated below, 2.15 g of precipitated amorphous atorvastatin, (from Example 2) and 97.9 g of the diluent. The ingredients were blended 10 min. using a Turbula™ shaker-mixer. The blend was delumped by passing it through a stainless steel, #30 U.S. Standard sieve. The formulation was then blended 10 min. using the Turbula™. Lastly, 1.99 g of vegetable source, magnesium stearate (Mallinckrodt, St. Louis, MO) was added and the contents blended 3 min. using the Turbula™. Finished blend was analyzed for segregation as described in Example 5h, with the low and high flow settings for the fluidization and the samples weights and extraction volumes indicated below (when different than Example 5h). Results are reported in Table 2.

(a) The diluent was calcium phosphate dibasic, anhydrous unmilled (A-Tab™, Rhodia, Chicago Heights, IL). The fluidization flow settings were 5 and 11, for low and high, correspondingly.

(b) The diluent was spray dried lactose monohydrate (Foremost Farms USA, Rothschild, WI). The fluidization flow

settings were 4 and 11, for low and high, correspondingly.

(c) The diluent was compressible sucrose (D.C. & T.S. White Di-Pac, Tate & Lyle, Brooklyn, NY). The fluidization flow settings were 8 and 18, for low and high, correspondingly.

(d) The diluent was microcrystalline cellulose (Avicel PH-200, FMC BioPolymer, Co. Cork, Ireland). The fluidization flow settings were 4 and 9, for low and high, correspondingly. The analytical sample size was 1.3 g with an extraction volume of 100 mL

(e) The diluent was Lactose Monohydrate, Modified (Fast Flo 316, Foremost Farms USA, Rothschild, WI). The fluidization flow settings were 6 and 11, for low and high, correspondingly. The analytical sample size was 2.0 g with an extraction volume of 200 mL

(f) The diluent was granular mannitol (Mannogem 2080, SPI Polyols, New Castle, DE). The fluidization flow settings were 20 and 35, for low and high, correspondingly. The analytical sample size was 2.0 g with an extraction volume of 200 mL

Table 2. Results of fluidization testing for binary blends of precipitated atorvastatin and diluents.

| Example | Top Sample, % Intent | Middle Sample, % Intent | Bottom Sample, % Intent | Segregation Number |
|---|---|---|---|---|
| 6a | 99.0 | 96.8 | 89.5 | 0.10 |
| 6b | 102.8 | 102.3 | 84.0 | 0.20 |
| 6c | 86.6 | 78.8 | 70.3 | 0.21 |
| 6d | 109.6 | 89.7 | 79.3 | 0.33 |
| 6e | 100.1 | 97.3 | 104.4 | -0.04 |
| 6f | 109.6 | 62.2 | 57.9 | 0.68 |

Examples 5 and 6 show that a range of diluents provide a range of segregation numbers, which differ with drug form, in an unpredictable fashion.

## EXAMPLE 7

### PREPARATION AND UNIFORMITY TESTING OF ATORVASTATIN/DILUENT TABLETS

Tablets were prepared from each of the formulations 5a-5g using a Kilian T100 rotary tablet press (Kilian & Co., Bristol, PA) with the slight modification for 5e in terms of batch size (11.6 g of atorvastatin, 1075.0 g of microcrystalline cellulose and 11.0 g of magnesium stearate). Tablets were prepared using 1/4" standard round concave (SRC) plain faced tooling, using a partial set of four tools. Tablets were prepared with approximately 100 mg each. The tablet press was run at 37 rpm (shoe speed of 30 rpm). This corresponds to a rate of approximately 20,000 tablets per hour for the corresponding full set of nine tools. Tablets were sampled (at least three samples from each point) from the beginning, middle and end of the run (about 1 kg of sample was run in each case). Analyses of the tablets were carried out according to the following procedures: For sample (a), 7.5 mL of 0.05M ammonium acetate was added to 1 tablet then shaken for 8 minutes followed by further addition of 12.5 mL of acetonitrile and shaking for 12 minutes; for (b) one tablet was added to 20 mL of 1:1 (v:v) 0.05M ammonium acetate buffer (pH7.4):acetonitrile and shaken for 25 minutes, and (f), one tablet was added to 20 mL of 1:1 (v:v) 0.05M ammonium acetate buffer (pH 7.4):acetonitrile and shaken for 20 minutes; for (c), (d), and (e), 1 tablet was combined with 10 mL of 1:1 (v:v) 0.05M ammonium citrate buffer (pH 7.4):acetonitrile and shaken for 20 minutes. In each case, the solutions were filtered using a Gelman Acrodisc polytetrafluroroethylene membrane (0.45μm pore size), and analyzed using HPLC (Phenomenex, Ultremex C18 column, 25.0 cm X 4.6 mm, HPLC HP 1100 series, 20 μL injection volume, flow of 1.5 mL/minutes; mobile phase of 53:27:20 (v:v:v) 0.05M ammonium citrate (pH 4.0):acetonitrile:tetrahydrofuran; detection at 244 nm). Results are reported in Table 3.

Table 3 Potency consistency for production of tablets with atorvastatin in the absence of a granulation step.

| Example | Blend Source (Example) | Start of Run, % Intent | Middle of Run, % Intent | End of Run, % Intent | Overall %RSD |
|---|---|---|---|---|---|
| 7a | 5a | 109.5 ± 8.4 | 94.9 ± 2.4 | 96.4 ± 3.5 | 8.6 |

(continued)

| Example | Blend Source (Example) | Start of Run, % Intent | Middle of Run, % Intent | End of Run, % Intent | Overall %RSD |
|---|---|---|---|---|---|
| 7b | 5b | 92.7 ± 2.2 | 101.0 ± 1.2 | 93.9 ± 0.8 | 4.3 |
| 7c | 5c | 93.1 ± 0.3 | 94.6 ± 2.0 | 95.8 ± 1.5 | 1.8 |
| 7d | 5d | 89.2 ± 1.6 | 92.0 ± 2.2 | 94.1 ± 0.7 | 2.8 |
| 7e | 5e (different batch size) | 83.6 ± 1.3 | 92.5 ± 0.5 | 97.1 ± 1.2 | 5.7 |
| 7f | 5f | 108.1 ± 4.6 | 89.7 ± 5.4 | 81.2 ± 0.6 | 13.3 |

Combining the data from Example 5 (segregation number) and Example 7 (tablet potency variability) shows a strong correlation, as seen in Figure 1. This correlation, with atorvastatin, allows for the determination of appropriate segregation numbers as a means of selecting appropriate diluents for production of atorvastatin dosage forms without a granulation step.

## Claims

**1.** A tablet comprising:

(a) 1-40% weight:weight amorphous atorvastatin, or a pharmaceutically acceptable salt thereof;
(b) 5-10% disintegrant;
(c) 0-10% binder;
(d) 0.5-2% lubricant; and
(e) the remainder diluent;

said tablet being prepared without a granulation step by compression;
with the proviso that the tablet does not contain approximately 1.2% to less than 5% concentration of an alkali metal salt additive.

**2.** The tablet according to claim 1 wherein said disintegrant is selected from the group consisting of carboxymethylcellulose, hydroxyproyl cellulose (low-substituted), microcrystalline cellulose, powdered cellulose, colloidal silicon dioxide, croscarmellose sodium, crospovidone, magnesium aluminum silicate, methylcellulose, polacrilin potassium, povidone, sodium alginate, sodium starch glycolate and starches.

**3.** The tablet according to claim 1 wherein said binder is selected from the group consisting of acacia, carboxymethylcellulose, hydroxyethylcellulose, hydroxypropylcellulose, dextrin, gelatin, guar gum, hydroxypropyl methylcellulose, magnesium aluminum silicate, maltodextrin, methylcellulose, polyethylene oxide, polymethacrylates, povidone, sodium alginate, starches and zein.

**4.** The tablet according to claim 1 wherein said lubricant is selected from the group consisting of calcium stearate, glyceryl palmitostearate, magnesium oxide, poloxamer, polyethylene glycol, polyvinyl alcohol, sodium benzoate, sodium lauryl sulfate, sodium stearyl fumarate, stearic acid, talc, zinc stearate and magnesium stearate.

**5.** The tablet according to claim 1 wherein the diluent comprises greater or equal to 50 wt% of the excipients.

**6.** The tablet according to claim 1 or claim 5 wherein said diluent is selected from the group consisting of calcium phosphate, calcium sulfate, carboxymethylcellulose calcium, cellulose, cellulose acetate, dextrates, dextrin, dextrose, fructose, glyceryl palmitostearate, hydrogenated vegetable oil, kaolin, lactitol, lactose, magnesium carbonate, magnesium oxide, maltitol, maltodextrin, maltose, polymethacrylates, pregelatinized starch, silicified microcrystalline cellulose, sodium chloride, sorbitol, starch, sucrose and talc.

**7.** The tablet according to claim 6 wherein said diluent is selected from the group consisting of large particle size lactose monohydrate, large particle size lactose anhydrous, large particle size microcrystalline cellulose and sodium chloride.

8. The tablet according to claim 1 comprising 0-5% weight:weight of an alkaline earth metal salt.

9. The use of the tablet according to any one of claims 1-8 for the preparation of a medicament for the treatment of hypercholesterolemia and/or hyperlipidemia, osteoporosis, benign prostatic hyperplasia or Alzheimer's disease.

**Patentansprüche**

1. Tablette, umfassend:

   (a) 1-40%, Gewicht:Gewicht, amorphes Atorvastatin oder ein pharmazeutisch verträgliches Salz davon;
   (b) 5-10% Zerfallsmittel;
   (c) 0-10% Bindemittel;
   (d) 0,5-2% Gleitmittel und
   (e) als den Rest Verdünnungsmittel;

   wobei die Tablette ohne einen Granulierungsschritt durch Verpressen hergestellt wird;
   mit der Maßgabe, dass die Tablette kein Alkalimetallsalzadditiv in einer Konzentration von etwa 1,2% bis weniger als 5% enthält.

2. Tablette nach Anspruch 1, wobei das Zerfallsmittel aus der Gruppe, bestehend aus Carboxymethylcellulose, Hydroxypropylcellulose (geringsubstituiert), mikrokristalliner Cellulose, gepulverter Cellulose, kolloidalem Siliciumdioxid, Croscarmellose-Natrium, Crospovidon, Magnesiumaluminiumsilikat, Methylcellulose, Polacrilin-Kalium, Povidon, Natriumalginat, Natriumstärkeglykolat und Stärken, ausgewählt ist.

3. Tablette nach Anspruch 1, wobei das Bindemittel aus der Gruppe, bestehend aus Acacia, Carboxymethylcellulose, Hydroxyethylcellulose, Hydroxypropylcellulose, Dextrin, Gelatine, Guargummi, Hydroxypropylmethylcellulose, Magnesiumaluminiumsilikat, Maltodextrin, Methylcellulose, Polyethylenoxid, Polymethacrylaten, Povidon, Natriumalginat, Stärken und Zein, ausgewählt ist.

4. Tablette nach Anspruch 1, wobei das Gleitmittel aus der Gruppe, bestehend aus Calciumstearat, Glycerylpalmitostearat, Magnesiumoxid, Poloxamer, Polyethylenglykol, Polyvinylalkohol, Natriumbenzoat, Natriumlaurylsulfat, Natriumstearylfumarat, Stearinsäure, Talk, Zinkstearat und Magnesiumstearat, ausgewählt ist.

5. Tablette nach Anspruch 1, wobei das Verdünnungsmittel 50 Gew.-% oder mehr der Exzipientien umfasst.

6. Tablette nach Anspruch 1 oder Anspruch 5, wobei das Verdünnungsmittel aus der Gruppe, bestehend aus Calciumphosphat, Calciumsulfat, Carboxymethylcellulose-Calcium, Cellulose, Celluloseacetat, Dextraten, Dextrin, Dextrose, Fructose, Glycerylpalmitostearat, hydriertem Pflanzenöl, Kaolin, Lactid, Lactose, Magnesiumcarbonat, Magnesiumoxid, Maltit, Maltodextrin, Maltose, Polymethacrylaten, vorgelatinierter Stärke, silizifizierter mikrokristalliner Cellulose, Natriumchlorid, Sorbit, Stärke, Saccharose und Talk, ausgewählt ist.

7. Tablette nach Anspruch 6, wobei das Verdünnungsmittel aus der Gruppe, bestehend aus Lactose-Monohydrat mit großer Partikelgröße, wasserfreier Lactose mit großer Partikelgröße, mikrokristalliner Cellulose mit großer Partikelgröße und Natriumchlorid, ausgewählt ist.

8. Tablette nach Anspruch 1, die 0 bis 5%, Gewicht:Gewicht, eines Erdalkalimetallsalzes umfasst.

9. Verwendung der Tablette nach einem der Ansprüche 1 bis 8 für die Herstellung eines Medikaments zur Behandlung von Hypercholesterinämie und/oder Hyperlipidämie, Osteoporose, gutartiger Prostatahyperplasie oder Alzheimer-Erkrankung.

**Revendications**

1. Comprimé comprenant :

   (a) 1-40% poids:poids d'atorvastatine amorphe, ou d'un sel pharmaceutiquement acceptable correspondant ;

(b) 5-10% de désintégrant ;
(c) 0-10% de liant
(d) 0,5-2% de lubrifiant ; et
(e) un diluant comme complément ;

ledit comprimé étant préparé sans étape de granulation par compression ;
étant entendu que le comprimé ne contient pas une concentration comprise entre 1,2% et moins de 5% d'un sel de métal alcalin comme additif.

**2.** Comprimé selon la revendication 1, dans lequel ledit désintégrant est sélectionné dans le groupe consistant en la carboxyméthylcellulose, l'hydroxypropyl cellulose (faiblement substituée), la cellulose microcristalline, la cellulose pulvérulente, le dioxyde de silicium colloïdal, la croscarmellose sodique, la crospovidone, le silicate de magnésium et d'aluminium, la méthylcellulose, la polacriline potassique, la povidone, l'alginate de sodium, l'amidon glycolate de sodium et les amidons.

**3.** Comprimé selon la revendication 1, dans lequel ledit liant est sélectionné dans le groupe consistant en l'acacia, la carboxyméthylcellulose, l'hydroxyéthyl cellulose, l'hydroxypropylcellulose, la dextrine, la gélatine, la gomme de guar, l'hydroxypropyl méthylcellulose, le silicate de magnésium et d'aluminium, la maltodextrine, la méthylcellulose, le poly(oxyde d'éthylène), les polyméthacrylates, la povidone, l'alginate de sodium, les amidons et la zéine.

**4.** Comprimé selon la revendication 1, dans lequel ledit lubrifiant est sélectionné dans le groupe consistant en le stéarate de calcium, le palmitostéarate de glycéryle, l'oxyde de magnésium, le poloxamère, le polyéthylène glycol, le poly(alcool vinylique), le benzoate de sodium, le lauryl sulfate de sodium, le stéaryl fumarate de sodium, l'acide stéarique, le talc, le stéarate de zinc et le stéarate de magnésium.

**5.** Comprimé selon la revendication 1, dans lequel le diluant représente une quantité égale ou supérieure à 50% en poids des excipients.

**6.** Comprimé selon la revendication 1 ou la revendication 5, dans lequel le diluant est sélectionné dans le groupe consistant en le phosphate de calcium, le sulfate de calcium, la carboxyméthylcellulose calcique, la cellulose, l'acétate de cellulose, les dextrates, la dextrine, le dextrose, le fructose, le palmitostéarate de glycéryle, l'huile végétale hydrogénée, le kaolin, le lactitol, le lactose, le carbonate de magnésium, l'oxyde de magnésium, le maltitol, la maltodextrine, le maltose, les polyméthacrylates, l'amidon prégélatinisé, la cellulose microcristalline silicifiée, le chlorure de sodium, le sorbitol, l'amidon, le saccharose et le talc.

**7.** Comprimé selon la revendication 6, dans lequel ledit diluant est sélectionné dans le groupe consistant en le lactose monohydraté à grande taille de particules, le lactose anhydre à grande taille de particules, la cellulose microcristalline à grande taille de particules et le chlorure de sodium.

**8.** Comprimé selon la revendication 1, comprenant 0-5% poids:poids d'un sel de métal alcalino-terreux.

**9.** Utilisation du comprimé selon l'une quelconque des revendications 1 à 8 pour la préparation d'un médicament pour le traitement de l'hypercholesterolémie et/ou de l'hyperlipémie, de l'ostéoporose, le l'hyperplasie bénigne de la prostate ou de la maladie d'Alzheimer.

FIG. 1

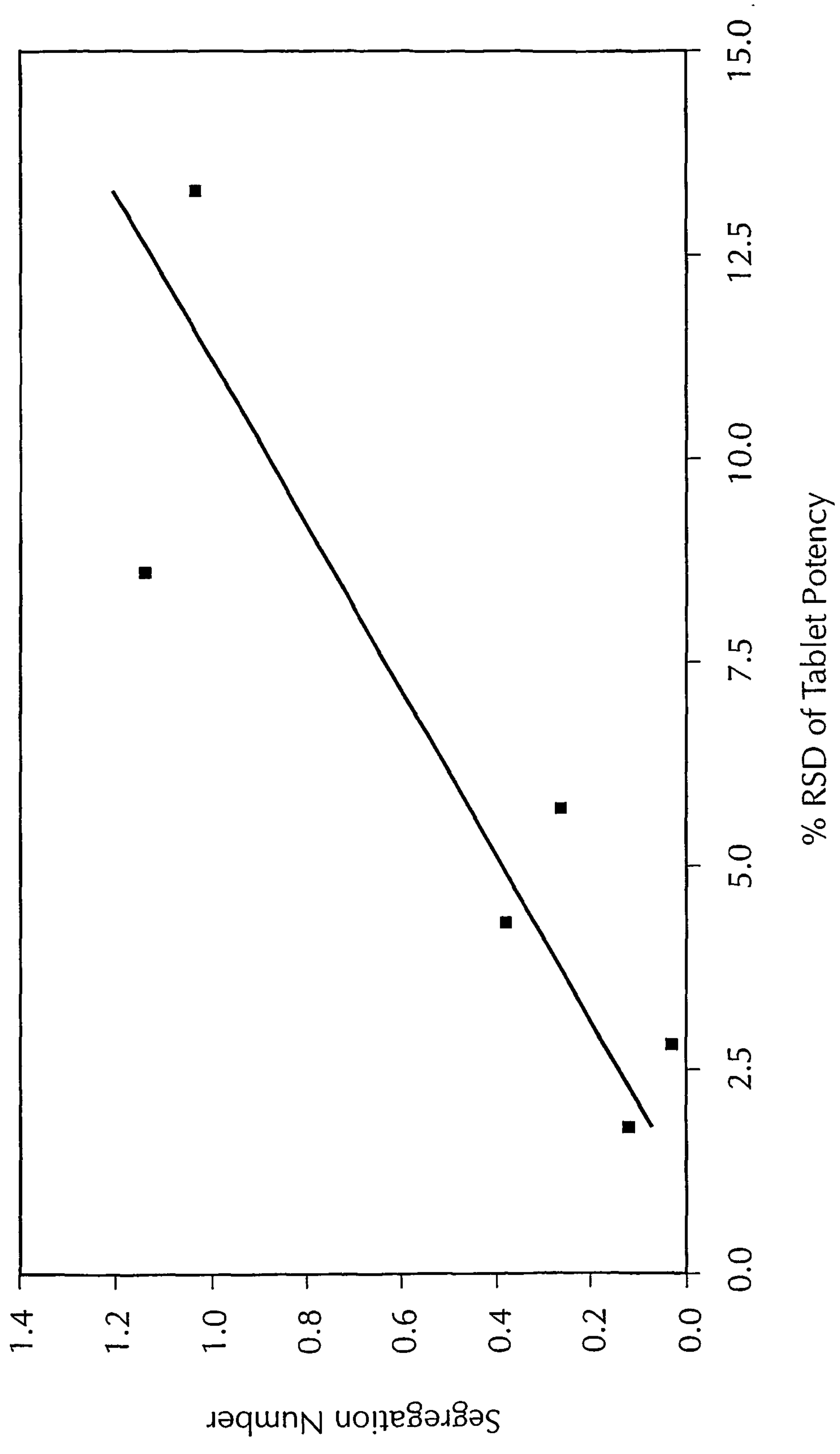

Segregation Number
1.4
1.2
1.0
0.8
0.6
0.4
0.2
0.0
% RSD of Tablet Potency
0.0
2.5
5.0
7.5
10.0
12.5
15.0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 47791803 P
- US 5273995 A
- US 4681893 A
- US 5003080 A
- US 5097045 A
- US 5103024 A
- US 5124482 A
- US 5149837 A
- US 5155251 A
- US 5216174 A
- US 5245047 A
- US 5248793 A
- US 5280126 A
- US 5397792 A
- US 5342952 A
- US 5298627 A
- US 5446054 A
- US 5470981 A
- US 5489690 A
- US 5489691 A
- US 5510488 A
- US 5686104 A
- US 5998633 A
- US 6087511 A
- US 6126971 A
- US 6433213 A
- US 6476235 A
- US 5969156 A
- US 6121461 A
- US 6605729 B
- WO 0071116 A
- WO 0128999 A
- WO 0136384 A
- WO 0142209 A
- WO 0241834 A
- WO 0243667 A
- WO 0243732 A
- WO 02051804 A
- WO 02057228 A
- WO 02057229 A
- WO 02057274 A
- WO 02059087 A
- WO 02083637 A
- WO 02083638 A
- WO 03011826 A
- WO 03050085 A
- WO 03070702 A
- WO 04022053 A
- US 2004024767 A
- US 20040253305 A
- WO 0035425 A
- WO 0176566 A1
- WO 9936060 A
- WO 03013608 A
- EP 1336405 A
- EP 20082003 A
- US 20050032880 A
- WO 3011826 A
- EP 1243524 A2
- EP 1241110 A1


### Non-patent literature cited in the description


- **KONNO, T.** *Chem. Pharm. Bull.,* 1990, vol. 38, 2003-2007
- **TAKEMOTO, M. ; NODE, K ; NAKAGAMI, H. ; LIAO, Y. ; GRIMM, M. ; TAKEMOTO, Y. ; KITAKAZE, M. ; LIAO, J.K.** *Journal of Clinical Investigation,* 2001, vol. 108 (10), 1429-1437
- Handbook of Pharmaceutical Excipients. Pharmaceutical Press, 2000
- **BERGE, S.M. et al.** Pharmaceutical Salts. *J. Pharm. Sci.,* 1977, vol. 66, 1